# EUROPEAN PATENT SPECIFICATION

(11) **EP 1 982 743 B1**
(45) Date of publication and mention of the grant of the patent: **19.06.2013**
(21) Application number: 08251255.9
(22) Date of filing: 31.03.2008
(51) Int. Cl.: A61M 25/02

(54) **Tube holder**
Schlauchhalter
Support de tubes

(30) Priority: 18.04.2007 GB 0707490
(43) Date of publication of application: 22.10.2008
(73) Proprietor: CLINIMED (Holdings) LIMITED, High Wycombe, Bucks HP10 9QY (GB)
(72) Inventor: Berry, Jeannette, Fetcham, Leatherhead Surrey, KT22 9GS (GB); Smith, Roy c/o Weland Medical Limited, Crawley West Sussex,RH10 2TU (GB)
(74) Representative: Townsend, Victoria Jayne

(56) References cited:
- DE-U1- 8 710 021
- US-A- 3 834 380
- US-A- 5 082 111
- US-A- 6 015 119
- US-A- 6 149 660

## Description

The present invention relates to a tube holder, for holding a tube such as a catheter or a drip to the body of a patient.

Tubes, such as catheters or those which pass to a patient from a drip, need to be attached to the body of a patient in such a way as to allow the patient to move whilst the tube remains attached. It is common to attach the tubes using adhesive bandages or plasters. To adjust the position of the tube the adhesive bandage would have to be removed and replaced. This often leads to poor positioning of the tube because any adjustment causes unnecessary inconvenience and discomfort to the patient and wastes bandages.

US 4165748 discloses a device which is adhesively fixed to patient but also has a Velcro^{™} "bridge" which removeably fastens around a tube, such as a catheter. Use of the removable fastener allows the tube's position to be adjusted without removing the device from the patient's skin. However, it does not offer the security of an adhesive fastening. It has been found that when the patient is unable to monitor the tube holder, for example when asleep, a patient can easily disturb the removable fastener and so the tube becomes detached from the patient's body.

US3834380 discloses a tube holder comprising a longitudinally split, elongate, cylindrical, clamping tube securely attached to an adhesively backed tape. A latching flap is closed about the clamping tube and a tube held therein, wherein the latching flap comprises interlocking means to securely hold the tube in place. This device does not allow a patient to move while the tube remains attached. If a users wishes to re-position the tube, the latching flap must be removed and the tube removed from the clamping tube. The device disclosed in US383480 does not allow for adjustment of the tube within the clamping tube before the tube is fixed by the latching flap and does not allow movement of the tube within the tube holder relative to the movement of a patient. If a patient moves about it is likely that the tube will be pulled from the holder.

The present invention sets out to provide a tube holder as further disclosed in claim 1 which alleviates the problems described above by providing a pad which can be either permanently or removeably fixed to a patient's body.

A tube holder for removeably securing a tube to the skin of a patient comprising a plastics film bonded to an adhesive material on a bodyside surface of the pad; and a separate first and second means for applying pressure to a tube located in the holder during use, wherein the first and second means are bonded to a tube-side surface of the plastics film and pressure can be applied, in use, to at least two opposing sides of the tube.

By "sides" it is meant areas on the circumference of the tube.

Applying pressure to at least two sides of the tube ensures the stability of the tube when it is held against a patient's skin, for example when the patient is moving around or when the patient is asleep and unable to monitor the tube.

Preferably, at least one of the means for applying pressure to the tube comprises at least one tab extending from the tube-side surface of the plastics film to form, in use, an arch over the tube.

By forming an arch it is meant that the at least one tab forms a bridge over the tube.

Preferably, at least one of the means for applying pressure to the tube comprises a layer of adhesive.

The layer of adhesive may comprise any one of an acrylic adhesive, a hot melt adhesive or a silicon adhesive.

More preferably, the layer of adhesive is pressure-sensitive.

Preferably, the layer of adhesive is covered by a removeable release liner.

More preferably, the release liner is a silicone coated paper.

Preferably, at least one of the means for applying pressure to the tube comprises a male/female connecting means.

Preferably, at least one of the means for applying pressure to the tube comprises a hook and loop fastener.

More preferably, the hook and loop fastener is Velcro^{™}.

A male/female connecting means for applying pressure to the tube, or a hook and loop fastener, for example Velcro^{™}, allows the tube holder to be adjusted during use.

The invention will now be described by way of example with reference to the accompanying diagrammatic drawings, in which :-
Figure 1 is a cross sectional view of a tube holder constructed in accordance with the present invention;
Figure 2 is a plan view from above of the tube holder of Figure 1; and
Figure 3 is a plan view from the side of the tube holder, in use, holding a tube.

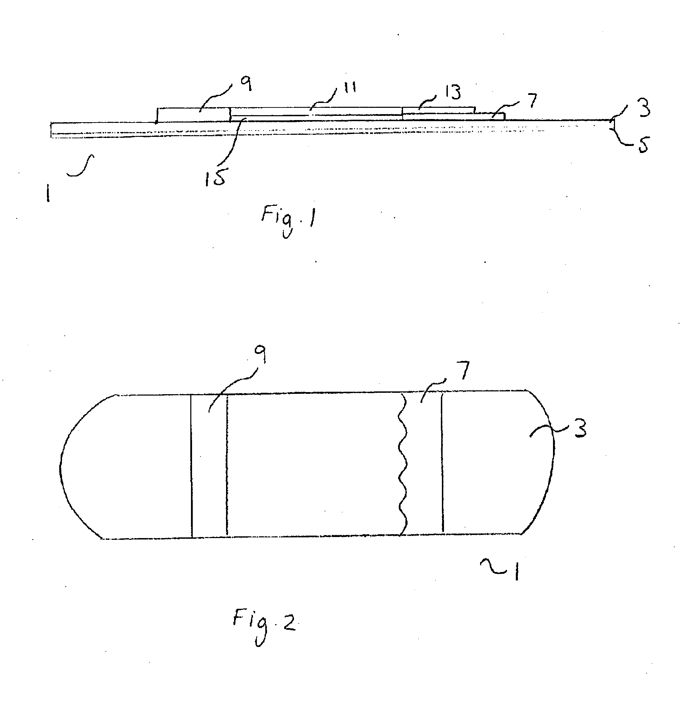

As shown in Figure 1, a tube holder 1 according to the invention comprises a thin adhesive pad 3. The dimensions of the pad 3 are chosen according to the tube with which it is to be used. For standard tubes the width of the pad may be 40mm and length 140mm or alternatively the width may be 25 mm and the length 110mm. The pad 3 is formed of a thin plastics layer which is coated with a pressure sensitive adhesive on a body side surface of the pad 3. The pressure sensitive adhesive may be an acrylic adhesive, a silicon adhesive or a hot melt adhesive. The pressure sensitive adhesive layer is temporarily covered and protected by the use of a release liner 5 made of a plastics film, such as a silicone coated paper to be removed immediately prior to use of the tube holder 1. The release line 5 may be disposed of after it is removed or it may remain connected to the tube holder after it is removed from the adhesive layer to be reusable to re-cover the adhesive layer.

Two tabs 7, 9 are fixed by an adhesive to the opposing tube side surface of the thin adhesive pad 3. The first tab 7 is shorter than the second tab 9. The first tab 7 is adhesively fixed along the full length of one of its surfaces to tube side surface of the pad 3. The opposing surface of the first tab 7 comprises a fastening surface. The fastening surface is a hook and loop fastener such as Velcro^{™}. The second tab 9 is a non-woven fabric such as polyurethane or an elastomeric material or a Lycra™-based polymer. Alternatively, the tab 9 may be made of a knitted fabric. The second tab 9 is fixed only along a minor portion of its length to the surface of the pad 3. The remaining length of the second tab comprises a section of hook and loop fastener 13 such as Velcro^{™}.

Between the first and second tabs 7,9 there is central section of the tube side surface of the pad 3 which comprises an adhesive layer temporarily covered and protected by a release liner 15 made of a plastics film.

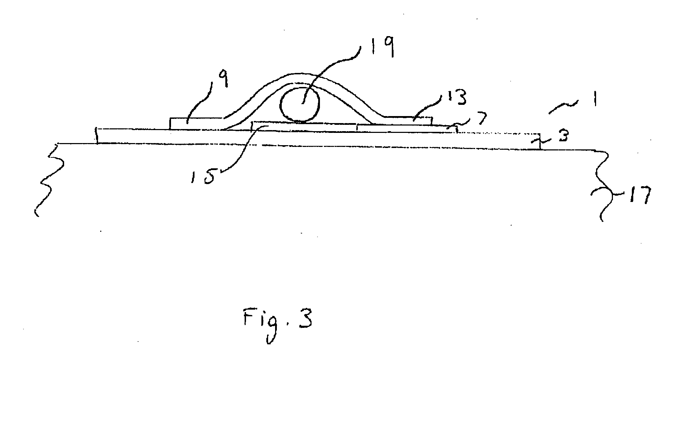

In use, as shown in Figure 3, the release liner 5 on the body side surface of the adhesive pad 3 is removed and the pad 3 is adhesively fixed to a user's skin 17. If a tube is to be temporarily held by the tube holder 1, the tube 19 is placed over the release liner 15 of the central section. The second longer tab 9 is then looped over the tube such that the Velcro^{™} section 13 of the second tab 9 meets and is removeably fixed to the Velcro^{™} section of the first shorter tab 7 to "bridge" the tube 19 and so hold the tube in place. If a user wishes to re-position the tube 19, the Velcro^{™} section 13 of the second tab 9 is pulled from the first tab 7 and the process, which is described above, is repeated.

If the tube 19 is to be permanently held by the tube holder 1 the release liner 5 on the body side surface of the adhesive pad 3 is removed and the pad 3 is adhesively fixed to a user's skin 17. The release liner 15 is removed from the central section of the tube side surface of the pad 3. The tube 19 is placed over and so adhesively fixed to the pad 3. The second longer tab 9 is then looped over to "bridge" the tube such that the Velcro^{™} section of the second tab 13 meets and is removeably fixed to the Velcro^{™} section of the first shorter tab 7 to further secure the tube 17 in place. If a user wishes to re-position the tube 19 the tube holder 1 would have to be removed from the user's skin and replaced.

The above described embodiment has been given by way of example only, and the skilled reader will naturally appreciate that many variations could be made thereto without departing from the scope of the present invention.

## Claims

1. A tube holder (1) for removeably securing a tube to the skin of a patient comprising a plastics film bonded to an adhesive material on a bodyside surface of the film; a separate first and second holding means bonded to a tube-side surface of the plastics film for applying pressure to a tube located in the holder during use, wherein the first holding means (7, 9, 13) comprises at least one tab extending from the tube-side surface of the plastics film to form, in use, an arch over a tube and **characterised in that** the second holding means comprises a layer of adhesive material and a removeable release liner (15) on the tube-side surface of the plastics film, wherein the release liner is removable to expose the adhesive layer thereunder, such that pressure is appliable, in use, to at least two opposing sides of the tube.

2. A tube holder (1) according to claim 1 wherein the adhesive material on the bodyside and/or the tube-side surface of the plastics film comprises an acrylic adhesive.

3. A tube holder (1) according to claim 1 wherein the adhesive material on the bodyside and/or the tube-side surface of the plastics film comprises a hot melt adhesive.

4. A tube holder (1) according to claim 1 wherein the adhesive material on the bodyside and/or the tube-side surface of the plastics film comprises silicon adhesive.

5. A tube holder (1) according to any preceding claim wherein the adhesive material on the bodyside and/or the tube-side surface of the plastics film is pressure-sensitive.

6. A tube holder (1) according to any preceding claim wherein the adhesive material on the body-side surface is covered by a removeable release liner (5).

7. A tube holder according to any preceding claim wherein the or each release liner (5, 15) is a silicone coated paper.

8. A tube holder (1) according to any preceding claim wherein the first holding means comprises a male/female connecting means.

9. A tube holder according to any of claims 1 to 8 wherein the first holding means (7, 9, 13) comprises a hook and loop fastener (13).

10. A tube holder (1) according to claim 10 wherein the hook and loop fastener (13) is Velcro™.

## Patentansprüche

1. Ein Schlauchhalter (1) zum entfernbaren Sichern eines Schlauches an der Haut eines Patienten, umfassend eine Kunststofffolie, aufgeklebt auf ein haftendes Material auf einer körperseitigen Oberfläche der Folie; separate erste und zweite Halterungsmittel, aufgeklebt auf einer schlauchseitigen Oberfläche der Kunststofffolie zum Aufbringen von Druck auf einen Schlauch, der sich während des Gebrauchs in dem Halter befindet, wobei das erste Halterungsmittel (7, 9, 13) wenigstens eine Schlaufe umfasst, die sich von der schlauchseitigen Oberfläche der Kunststofffolie erstreckt, um, im Gebrauch, einen Bogen über einen Schlauch zu bilden und **gekennzeichnet dadurch, dass** das zweite Halterungsmittel eine Schicht von haftendem Material und ein entfernbares Trennpapier (15) auf der schlauchseitigen Oberfläche der Kunststofffolie umfasst, wobei das Trennpapier entfernbar ist, um die haftende Schicht darunter freizulegen, so dass, im Gebrauch, Druck auf wenigstens zwei entgegengesetzte Seiten des Schlauches aufbringbar ist.

2. Ein Schlauchhalter (1) nach Anspruch 1, wobei das haftende Material auf der körperseitigen und/oder der schlauchseitigen Oberfläche der Kunststofffolie ein Acrylhaftmittel umfasst.

3. Ein Schlauchhalter (1) nach Anspruch 1, wobei das haftende Material auf der körperseitigen und/oder der schlauchseitigen Oberfläche der Kunststofffolie einen Schmelzklebstoff umfasst.

4. Ein Schlauchhalter (1) nach Anspruch 1, wobei das haftende Material auf der körperseitigen und/oder der schlauchseitigen Oberfläche der Kunststofffolie Silikonhaftmittel umfasst.

5. Ein Schlauchhalter (1) nach einem der vorangehenden Ansprüche, wobei das haftende Material auf der körperseitigen und/oder der schlauchseitigen Oberfläche der Kunststofffolie selbstklebend ist.

6. Ein Schlauchhalter (1) nach einem vorangehenden Anspruch, wobei das haftende Material auf der körperseitigen Oberfläche mit einem entfernbaren Trennpapier (5) bedeckt ist.

7. Ein Schlauchhalter nach einem vorangehenden Anspruch, wobei das oder jedes Trennpapier (5, 15) ein silikonüberzogenes Papier ist.

8. Ein Schlauchhalter (1) nach einem vorangehenden Anspruch, wobei das erste Halterungsmittel ein männlich/weibliches Verbindungsmittel umfasst.

9. Ein Schlauchhalter nach einem der vorangehenden Ansprüche 1 bis 8, wobei das erste Halterungsmittel (7, 9, 13) einen Klettverschluss (13) umfasst.

10. Ein Schlauchhalter (1) nach Anspruch 9, wobei der Klettverschluss (13) Velcro^{™} ist.

## Revendications

1. Support de tube (1) pour fixer de manière amovible un tube à la peau d'un patient, comprenant un film plastique collé à un matériau adhésif sur une surface du film côté corps ; des premier et deuxième systèmes de support séparés liés à une surface côté tube du film plastique pour appliquer une pression sur un tube situé dans le support pendant l'utilisation, dans lequel le premier système de support (7, 9, 13) comprend au moins une patte s'étendant depuis la surface côté tube du film plastique pour former, en cours d'utilisation, un arc sur un tube et **caractérisé en ce que** le deuxième système de support comprend une couche de matériau adhésif et un revêtement de décollement amovible (15) sur la surface côté tube du film plastique, dans lequel le revêtement de décollement est amovible pour exposer la couche adhésive située en dessous de celle-ci, de sorte qu'une pression soit applicable, en cours d'utilisation, à au moins deux côtés opposés du tube.

2. Support de tube (1) selon la revendication 1, dans lequel le matériau adhésif sur la surface côté corps et/ou côté tube du film plastique comprend un adhésif acrylique.

3. Support de tube (1) selon la revendication 1, dans lequel le matériau adhésif sur la surface côté corps et/ou côté tube du film plastique comprend un adhésif thermofusible.

4. Support de tube (1) selon la revendication 1, dans lequel le matériau adhésif sur la surface côté corps et/ou côté tube du film plastique comprend un adhésif au silicium.

5. Support de tube (1) selon l'une quelconque des revendications précédentes, dans lequel le matériau adhésif sur la surface côté corps et/ou côté tube du film plastique est sensible à la pression.

6. Support de tube (1) selon l'une quelconque des revendications précédentes, dans lequel le matériau adhésif sur la surface côté corps est recouvert d'un revêtement de décollement amovible (5).

7. Support de tube selon l'une quelconque des revendications précédentes, dans lequel le ou chaque revêtement de décollement (5, 15) est un papier revêtu de silicium.

8. Support de tube (1) selon l'une quelconque des revendications précédentes, dans lequel le premier système de support comprend des moyens de connexion mâles/femelles.

9. Support de tube selon l'une quelconque des revendications 1 à 8, dans lequel le premier système de support (7, 9, 13) comprend une fixation à crochet et boucle (13).

10. Support de tube (1) selon la revendication 10, dans lequel la fixation à crochet et boucle (13) est un Velcro™.
